Europäisches Patentamt

**European Patent Office** (11) Publication number: **0 038 783**

Office européen des brevets **A1**

## EUROPEAN PATENT APPLICATION

(21) Application number: **81830059.2**

(22) Date of filing: **14.04.81**

(51) Int. Cl.³: **A 61 B 5/00**
**A 61 B 10/00, G 01 F 1/52**

(30) Priority: **21.04.80 IT 6762480**

(43) Date of publication of application:
**28.10.81 Bulletin 81/43**

(84) Designated Contracting States:
**AT BE CH DE FR GB LI LU NL SE**

(71) Applicant: **Sguazzi, Angelo**
**Via Arnaldo da Brescia, 9**
**I-10134 Torino(IT)**

(72) Inventor: **Sguazzi, Angelo**
**Via Arnaldo da Brescia, 9**
**I-10134 Torino(IT)**

(74) Representative: **Jacobacci, Filippo et al,**
**c/o Jacobacci-CASETTA & PERANI S.p.A. Via Alfieri 17**
**I-10121 Torino(IT)**

(54) Apparatus for determining the rate of flow of urine.

(57) Apparatus for determining the rate of flow of urine includes an elongate container (10) provided with a series of adjacent, upwardly open receptacles (16) separated from each other by partitions (14), and a vertical column (12) located at one end of the container (10) and having a longitudinal, tubular cavity (18) for receiving the urine. The bottom of the tubular cavity (18) in the column (12) communicates through a discharge port (38) with an outflow tube (40) projecting into the said end of the container (10) and having its axis inclined upwardly and towards the opposite end of the container (10) at an angle of substantially 45° to the axis of the column (12).

./...

Croydon Printing Company Ltd.

FIG. 1

## Apparatus for determining the rate of
## flow of urine

The present invention relates to apparatus for determining the rate of flow of urine which can be used in the medical field for controlling and diagnosing infection of the urinary system.

Apparatus of this type conventionally used in urology generally consists of extremely complicated and expensive devices of considerable weight and bulk which are thus difficult to transport.

A proposal to avoid the disadvantages of the conventional apparatus and to provide a simple, economic device has been put forward in U.S. Patent number 2,648,981. The device described in this patent basically includes a container divided up by a series of vertical partitions into a plurality of compartments intended to receive the liquid; and a receptacle, for receiving the liquid flow, mounted on the upper part of the container and provided with a plurality of vertically-spaced, outlet orifices, corresponding in number to the number of the compartments of the container and each disposed so as to discharge liquid into a respective compartment. These orifices define different levels within the receptacle which are reached by the liquid according to the rate of the liquid flow itself. Each orifice represents a liquid flow determined by the volume of that part of the receptacle disposed below the orifice and by the dimensions of the lower orifices, whereby the maximum flow rate of the liquid during a predetermined period of time is indicated at the end of this time period by the number of compartments containing liquid.

This solution, although notably simpler and more functional than the conventional devices does not allow a sufficiently precise indication of the actual characteristics of the urine flow to be obtained. The indication which can be obtained by means of this apparatus is, in fact, simply a qualitative indication of the maximum urine flow rate as provided by the presence of liquid in the compartment of the container corresponding to the orifice of the receptacle situated at the highest level reached by the liquid in the receptacle. Clearly, the major part of the urine will always be discharged into the compartments corresponding to the orifices located at the lowest levels, rendering an exact quantitative analysis impossible at the end of the test.

The object of the present invention is to avoid the said disadvantage by providing apparatus for determining the urine flow rate which, as well as being simple and economic to manufacture and easy to transport, allows a precise quantitative indication of the urine flow rate to be obtained.

In order to achieve this object, the present invention provides apparatus for determining the urine flow rate, characterised in that it comprises an elongate container in the form of a substantially rectangular tub provided with a series of upwardly open, transverse receptacles disposed side-by-side and separated from each other by parallel, vertical partitions, and a vertical column located at one end of the container and having a longitudinal tubular cavity for receiving the urine, the said column having a discharge port at its lower end through which the bottom of the tubular cavity communicates with an outflow tube projecting into the said one end of the container and having its axis inclined upwardly and towards the opposite ends of the container; each one of the said receptacles corresponding to a predetermined liquid flow rate whereby in use, the urine poured into the tubular cavity of the column spouts from the said inclined outflow tube with a speed which is dependent on the liquid head within the said tubular cavity and reaches substantially uniquely that receptacle corresponding to the value of the urine flow rate of the user.

Further characteristics of the invention will become clear in the course of the following detailed description, with reference to the appended drawings, provided purely by way of non-limiting example, in which:

Figure 1 is a schematic perspective view of apparatus according to the invention,

Figure 2 is a longitudinal sectional view, on an enlarged scale, taken on line II-II of Figure 1, and

Figure 3 is an exploded perspective view, on an enlarged scale, of a detail illustrated in Figures 1 and 2.

Referring to the drawings, the apparatus according to the invention basically consists of a container 10 and a vertical, tubular column 12 fixed at its lower end to one end of the container 10.

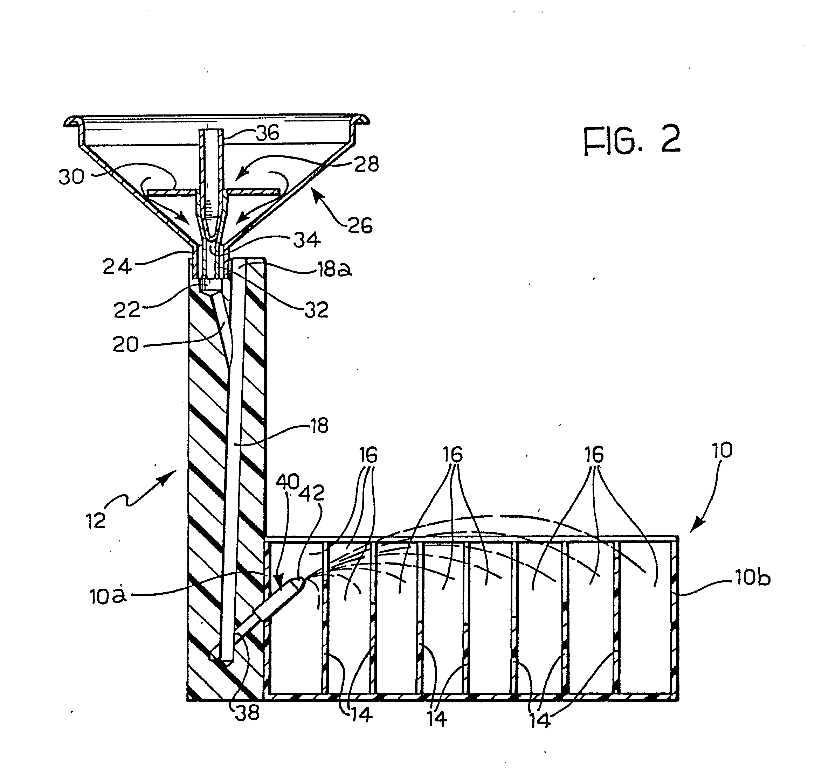

The container 10 is constituted by a rectangular tub, preferably of plastics material, divided internally by a series of rigid partitions 14 disposed parallel to the shorter walls 10a, 10b of the container 10 and sealingly connected to the longer walls 10c, 10d of the container 10. As is clearly shown in Figure 2, the dividing partitions 14 located in the central part of the container 10 are lower in height than the partitions 14 adjacent the walls 10a and 10b of the container 10. The partitions 14 between the central ones and the end ones have intermediate heights.

The partitions 14 define, together with the walls of the container 10, a plurality of adjacent upwardly-open, transverse receptacles 16.

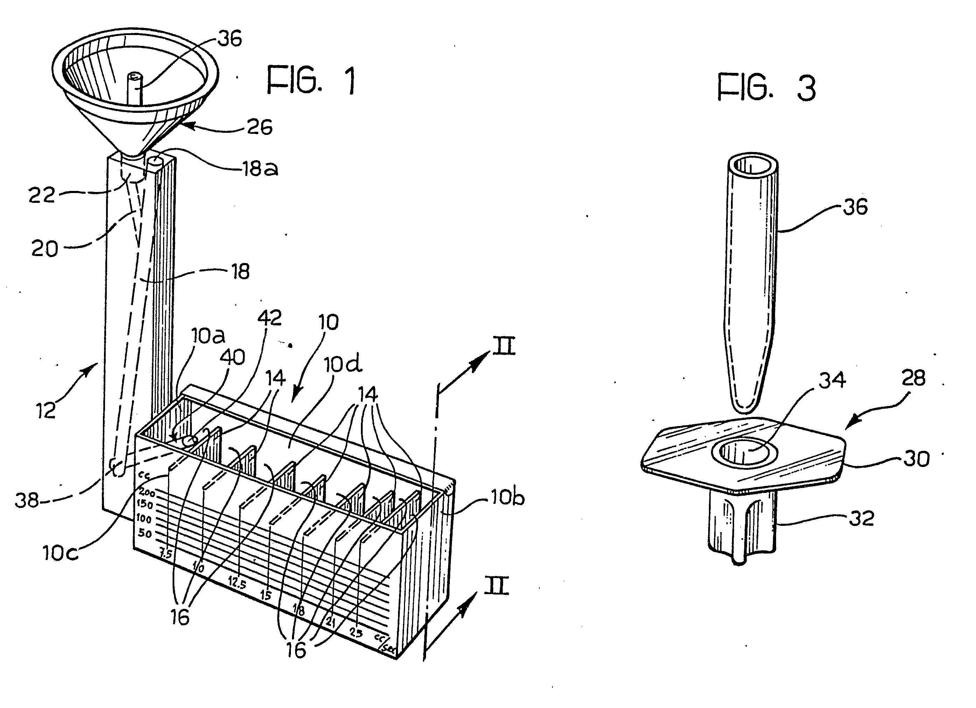

One of the longer walls of the container 10, for example, the wall 10c, is formed of transparent material and, as illustrated in Figure 1, bears a series of indications of the quantity and volumetric flow rate of the urine, in order to facilitate the analysis of the results at the end of the tests. The indications relating to quantity are constituted by horizontal level lines located above each other and each corresponding to a predetermined volumetric quantity, while the indications relating to the liquid flow rate are constituted by predetermined reference values associated each with one of the receptacles 16. The receptacle 16 adjacent the wall 10a of the container 10 corresponds to a minimum predetermined flow rate, for example 7.5 cm$^3$ per second, while the receptacle 16 adjacent the other shorter wall 10b of the container 10 corresponds to a maximum predetermined value of the flow rate, for example, greater than 25 cm$^3$ per second.

The receptacles 16 can either be formed directly within the container 10 or can alternatively be constituted by disposable flexible bags (that is, bags which can be thrown away after use) releasably fixed to the longer walls 10c, 10d of the container 10.

The column 12 is constituted, in the example illustrated, by an elongate element of plastics material rigidly fixed at its lower end to the shorter wall 10a of the container 10. The column 12 has a central longitudinal tubular cavity 18 which extends from its upper end to a short distance from its bottom end. At its upper end, the tubular cavity 18 communicates, a short distance from the upper end of the column 12, with a passage 20 which opens into the bottom of a hole 22 formed in the upper face of the column 12. Into the hole 22 is inserted the pipe 24 of a vertical-axis funnel 26 which is provided to receive the urine and convey it into the tubular cavity 18. Within the funnel 26, which may, to advantage, be moulded in a single piece with the column 12, is inserted a deflector element 28 illustrated in detail in Figure 3. The deflector element 28, which is provided to prevent the direct entry of urine into the passage 20 by diverting its flow tangentially to the inner wall of the narrower part of the funnel 26, basically comprises an upper horizontal plate 30 and a lower, centering member 32. The plate 30 has a polygonal profile the vertices of which rest upon the internal surface of the funnel so as to define a series of peripheral passages for the liquid. The centering member 32 is inserted coaxially within the pipe 24 and is shaped so as to bear against the internal surface of this pipe 24 and define a series of axial passages for the liquid.

The deflector element 28 is to advantage provided at its upper end with an axial bore 34 which constitutes a seat for a pipette 36 which may be used for taking up a sample of urine to be analysed.

The upper end of the tubular cavity 18, indicated by 18a, is open to the atmosphere and constitutes a vent passage to prevent air being entrained in the liquid flow in use, thus avoiding undesirable bubbling phenomena.

Close to the bottom of the tubular cavity 18, the column 12 has an outflow port 38 which places the tubular cavity 18 into communication with an outflow tube 40 which projects laterally from the column 12 into the container 10, in the zone between the wall 10a and the dividing partition 14 adjacent thereto. The outflow tube 40, which is provided at its outer end with a nozzle of predetermined bore, 42, has its axis inclined upwardly and towards the wall 10b of the container at an angle of substantially 45$^{o}$ to the axis of the column 12.

In use, the apparatus according to the invention allows the characteristics of the urine flow of the user to be examined simply and rapidly, by providing an immediate visual indication, through the references carried by the wall. 10c of the container 10, of the values both of the flow rate and of the total quantity of the urine. In fact, in use, the urine poured into the tubular cavity 18 of the column 12 through the funnel 26 spouts from the outlet end 42 of the outflow tube 40 at a velocity which depends on the head of liquid within the cavity 18 so as to reach, apart from short transistory initial and final periods, a unique receptacle 16. The difference in height between the partitions 14 of the container 10 determines, together with the distances between these partitions 14, the flow necessary for the jet to reach the receptacle 16 corresponding to the real value of the urine flow rate of the user.

From the above, it is clear that the apparatus according to the invention is extremely simple, functional and economic, is easy to transport and may be used directly by a patient without the intervention or the presence of specialised personnel.

Naturally, the principle of the invention remaining the same, the details of construction and the embodiments may be varied widely with respect to that described and illustrated without thereby departing from the scope of the present invention.

## CLAIMS

1. Apparatus for determining the rate of flow of urine, characterised in that it comprises an elongate container substantially in the form of a rectangular tub (10) provided with a series of transverse upwardly-open receptacles (16) disposed side by side and separated from each other by vertical partitions (14) parallel to each other, and a vertical column (12) disposed. at one end (10a) of the container and provided with a longitudinal tubular cavity (18) for receiving the urine, said column having in its lower part a discharge port (38) through which the bottom of said tubular cavity communicates with an outflow tube (40) projecting into said one end of the container and having its axis inclined upwardly and towards the opposite end (10b) of the container with respect to the axis of the column; each one of the said receptacles (16) corresponding to a predetermined flow of liquid whereby in use the urine poured into the tubular cavity of the column spouts from the said outflow tube (40) with a speed which depends upon the liquid head within the tubular cavity and reaches substantially uniquely that one of the said receptacles which corresponds to the rate of flow of urine of the user.

2. Apparatus according to Claim 1, characterised in that the tubular cavity (18) of the column (12) communicates in its upper part with a vent passage (18a).

3. Apparatus according to Claim 1, characterised in that the column (12) is provided at its upper end with a funnel portion for receiving the urine and conveying it into the tubular cavity (18) of the column.

4. Apparatus according to Claim 1, characterised in that the axis of the said outflow tube (40) is inclined with respect to the axis of the column at an angle substantially corresponding to 45°.

5. Apparatus according to Claim 1, characterised
in that the said receptacles (16) of the container
(10) are directly defined by the zones of the container
between the walls (10a, 10b, 10c, 10d) of said container
and the said vertical partitions (14).

6. Apparatus according to Claim 1, characterised
in that the said receptacles (16) are formed by flexible
disposable bags supported in a removable way by the
container.

7. Apparatus according to Claim 1, characterised in
that the height of the vertical partitions    (14) in
the central part of the container (10) is less than the
height of the vertical partitions disposed adjacent
the opposite ends of the container, the partitions located
between the central and end partitions having intermediate
heights.

8. Apparatus according to Claim 1, characterised in
that at least one of the longitudinal walls (10c) of
the container is transparent.

9. Apparatus according to Claim 8, characterised in
that the said at least one transparent wall (10c) of
the container is provided with reference indications
concerning the amount and the volume flow of the urine.

10. Apparatus according to Claim 3, characterised in
that the said funnel portion (26) of the column (12)
is provided, within its cavity and adjacent to its
narrow section (24), with a baffle element (28)
comprising a horizontal plate (30) having a polygonal
profile the vertices of which rest upon the internal
surface of the funnel portion, the said plate supporting
a centering member (32) extending coaxially within
the pipe (24) of the funnel and defining with the
internal surface of the said pipe of the funnel a
series of axial passages for the liquid.

11. Apparatus according to Claim 10, characterised in that the said baffle element (30) is provided with an upwardly-open axial seat (34) within which a pipette is removably inserted, the said pipette extending within the cavity of the said funnel portion (26) and being usable for taking urine samples.

FIG. 1

FIG. 3

1/2

0038783

FIG. 2

# 0038783

## EUROPEAN SEARCH REPORT

Application number

EP 81 83 0059

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| | US - A - 2 648 981 (W.M. DRAKE, Jr)<br>* Column 1, lines 27-40; column 2, lines 20-52; column 3, line 28 - column 4, line 48; figures 1-7 *<br>-- | 1,3,5, 10 | A 61 B  5/00<br>10/00<br>G 01 F  1/52 |
| | US - A - 3 871 231 (A.J. CIARICO/ THE KENDALL CO.)<br>* Abstract; column 2, lines 1-22; column 2, line 60 - column 3, line 52; column 5, lines 10-27; column 5, line 64 - column 6, line 5; figures 1-8 *<br>-- | 1,3,6, 8-10 | |
| | US - A - 1 840 608 (C.S. SLICHTER)<br>* Page 1, line 61 - page 2, line 16; figures 1,2 *<br>-- | 1,9 | TECHNICAL FIELDS SEARCHED (Int. Cl.³)<br><br>A 61 B  5/00<br>10/00<br>G 01 F  1/52 |
| | US - A - 2 663 188 (S.N. NELSON/ AMERICAN VISCOSE CORP.)<br>* Column 1, lines 14-32; column 2, lines 5-26; column 2, line 49 - column 4, line 54; figures 1-6 *<br>-- | 1,4,9 | |
| | FR - A - 2 369 831 (MEDICAL DEVI-CES, INC.)<br>* Page 1, line 38 - page 2, line 24; page 3, line 1 - page 4, line 39; page 8, lines 10-20; figures 1-3 *<br>-- | 1,2,5, 6,8,9 | CATEGORY OF CITED DOCUMENTS<br><br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| | FR - A - 2 181 066 (M. TOMASELLO)<br>* Page 1, line 24 - page 2, line 33; figures 1-3 *<br>--- | 11 | &: member of the same patent family, corresponding document |

The present search report has been drawn up for all claims

| Place of search | Date of completion of the search | Examiner |
|---|---|---|
| The Hague | 30-06-1981 | RIEB |

EPO Form 1503.1  06.78